(19) Europäisches Patentamt

European Patent Office

Office européen des brevets

(11) EP 1 244 047 A2

(12) EUROPEAN PATENT APPLICATION

(43) Date of publication:
25.09.2002 Bulletin 2002/39

(51) Int Cl.7: G06F 19/00

(21) Application number: 02251970.6

(22) Date of filing: 20.03.2002

(84) Designated Contracting States:
AT BE CH CY DE DK ES FI FR GB GR IE IT LI LU
MC NL PT SE TR
Designated Extension States:
AL LT LV MK RO SI

(30) Priority: 20.03.2001 US 277237

(71) Applicant: Ortho Clinical Diagnostics Inc.
Rochester, New York 14626 (US)

(72) Inventor: Atkins, David
Summit, New Jersey 07901 (US)

(74) Representative: Mercer, Christopher Paul et al
Carpmaels & Ransford
43, Bloomsbury Square
London WC1A 2RA (GB)

(54) **Method for providing clinical diagnostic services**

(57) A method for providing clinical diagnostic services is provided. The method includes collecting a biological sample, analyzing the biological sample to determine at least a part of the composition of its genetic material, the behavior of the genetic material, or a protein, reporting the results of the analysis (e.g., to a health care provider), and incorporating information obtained through the analysis into subsequent analyses of biological samples. The information obtained from the analysis can. for example, be incorporated into subsequent analyses by using it to improve the algorithmic or database components of the information products used or can be used to improve the statistical reliability of the analyses. Database systems and devices for conducting these methods are also presented.

Figure 1

**Description**

**Background of the Invention**

The invention relates to the field of clinical diagnostics and laboratory medicine.

Genetically based diagnostics are rapidly becoming standard tools in clinical laboratories. These diagnostics attempt to correlate physiological condition, disease state, or the proclivity for disease with some aspect of genetic composition or the behavior of genetic material within an organism. This includes analyses based on the presence or absence of genetic mutations such as sequence insertions, deletions, or mismatches. It can also include information about the manner in which gene expression occurs within an individual or a part of an individual (e.g., a cell) such as whether certain expression is up-regulated or down-regulated.

The utility of the diagnostic methods is a function of the power of the bioinformatic systems used to make the correlations referred to above. Most of these bioinformatic systems require the user to submit a sequence (nucleotide bases or amino acids) in a prescribed format. The systems then engage algorithms to have the sequence compared to other known sequences or the genetic expression profile compared to other expression patterns. The similarity of known and sample sequences and profiles are then compared or "scored" according to a variety of rules. Where a sequence to which the unknown sample is compared is known to have some physiological effect or be representative of a condition or disease state, an unknown sample that is similar to the known sequences in the systems may be said to have that condition or disease state. Bioinformatic systems that use algorithms to analyze sequence similarities include BLAST and FASTA computer programs. The robustness of the databases used to compare genetic information from unknown samples with genetic information reflective of known conditions is important.

The algorithmic aspects of the bioinformatic systems also affect the utility of the diagnostics. The programming logic and statistical and mathematical relationships that are used to determine when one sequence is similar to another are central to the utility of these systems as an aid in making diagnostic and prognostic judgments. However, there is an even more fundamental biological component to bioinformatics; ascribing functionality to the identity and expression of the sequences. If the relationships between conditions of interest and genetic information were precisely known this would not be a perplexing problem. Of course, this is not the case. While some diseases or conditions arc known to correlate directly with certain genetic profiles, most are entirely unknown or are only incompletely known. The probability of properly assessing disease state or condition improves as more elements of the genetic profile associated with those conditions are determined. For example, p53 mutations are events frequently seen in certain cancers such as colorectal cancer but thus far, no specific p53 mutation or group of p53 mutations can be used to definitively diagnose colorectal cancer, *c.f.*, p53 as a *Murker for Colorectal Cancer*, Asco on Line, http://www.asco.org/prof/pp/html/m_tumor8.htm. Some have speculated that epigenetic changes such as DNA methylation may also have diagnostic or prognostic value related to colorectal cancer. *c.f.*, Pharoah and Caldas, *Molecular Geneties and the Assessment of Human Cancers*, Expert Reviews in Molecular Medicine, http://www-crmm.cbcu.cam.ac.uk/99000526h.htm. Thus, one might speculate further that the presence of both p53 mutations and DNA methylation at certain sites improves the probability of accurately diagnosing colorectal cancer. As additional profile elements are identified the databases and algorithms used to compare normal and diseased or affected genetic material must be updated to realize these improvements.

Diagnostic services are usually provided by laboratories at the direction or request of a health care provider. The laboratory receives the patient samples from the health care provider, then conducts diagnostic assays, attains results, and then communicates those results to the patient or to the health care provider. This model also applies to genetically based diagnostics such as those that are dependent on amplification of genetic material. As noted above, analysis of the results of genetically based tests involve algorithmic manipulations of robust databases. These algorithms may be periodically updated as new information about genetic profiles is obtained but this must wait until clinical information is sought and integrated into such information products. Thus, the process is bifurcated at best. In one aspect of the typical process, patient genetic material is analyzed. In a wholly separate aspect of the process the information products used in the analysis are created and made available to the party conducting the analysis. There is no way under such a process to continuously improve the robustness of the database, the power of the algorithm used to conduct the analysis, and the confidence interval of the results obtained from the process.

Artificial neural networks (ANNs) have been proposed as one method for creating powerful algorithms for processing diagnostic information. U.S. patent 6,058,322 to Nishikawa and U.S. Patent 5,769,074 to Barnhill are examples. ANNs do not resolve the existing problems.

ANNs such as those described by Barnhill compare a variety of data to a network that has been trained to ascribe significance to each data component. For example, if one were analyzing a sample to diagnose prostate cancer. PSA and age might be two data elements that the network is trained to consider. The network might be trained so that a given PSA concentration at one age might be given more weight as an indicator of the presence of the cancer than the same PSA level at a different age.

These ANNs solve multi-variate problems by forming a multi-variable (weights) mathematical model on the basis of examples, and then applying their models to realistic cases. This process is generally referred to as training. The network itself can ultimately select the best rules to use to compare data. However, an ANN must be trained such that it meets prescribed statistical requirements (e.g., confidence level and positive predictive value) before it is ready to be used. In this sense, ANNs such as the one described in the Barnhill patent are static. There are discrete uses of data as training, testing, or sample cases. Training is not a continuous process.

Another distinguishing feature of the Bamhill patent is that the comparisons that it makes are of necessity based on "normal" values arrived at through statistical analysis as part of the training process. The act of training is itself an act of determining or setting normal ranges. Once trained, the ANN is queried to compare actual patient data to these normal values to assess diagnosis or prognosis. Aside from the algorithmic aspects of ANNs, this is rather standard treatment of data relating to, for example, clinical measurements of typical serum markers such as PSA. Without the ANN, a physician would merely compare the level of the marker with normal values provided in references. The power of the ANN is that it permits normal ranges to be configured such that they account for a number of variables that would be difficult for humans to simultaneously consider.

No ANN proposes a process that expands or contracts the number and/or significance of genetically related indicators (e.g. specific deletion sequences, epigenic mutations) to improve the relationship between the genetic profile and the diagnosis or prognosis during the clinical use of the diagnostic algorithm and database.

U.S. Patent 6,056,690 to Roberts proposes the use of Bayesian networks in constructing a diagnostic decision support tool. Bayesian networks are also called belief networks or causal probabilistic networks and use probability theory as an underpinning for reasoning under uncertainty. The ability of Bayesian networks to explain their reasoning is an important distinction over most ANNs. Despite this. Roberts does not propose improving the reasoning process itself as a function of the clinical use of the system.

U.S. Patent 5,966,711 to Adams proposes the use of autonomous intelligence agents to update databases and algorithms from a results table. The patent is directed to the structure of a system of algorithms and databases that interact with each other. In this system, updated components can communicate with the base systems when the base system needs assistance as, for example, when a sequence search reveals no close matches. The patent does not address validation of data that is used to form the daemon update programs nor does it address the source of the data. Without validation, operations that look to ever improving statistical reliability based on an increasing sample size can experience problems. For example, if the daemon program contained gene expression data that was not in the base system and was not validated its use would actually add to the uncertainty of the results generated. Moreover, the patent does not indicate that improvements in statistical reliability are even possible. This is because the daemons are used to interject only information and programming steps that were not previously present in the base system. There is no mention of using such daemons to reintroduce information that is already present thereby increasing the sample size from which statistical confidence is attained.

U.S. Patent 5,024,699 proposes the establishment of a system for inputting the results of patient testing and providing clinical advice to the patients based on them. The patent describes a process in which medicine dosage algorithms are modified based on those results. The algorithm in this case is one that is relevant only to the patient for whom the result was entered. It is not a systemic algorithm that affects the manner in which data is interpreted across the entire patient pool.

Methods for providing analytical diagnostic services that continually upgrade the power and utility of the information products used in providing those services would be beneficial. The ability to combine diagnostic information from a variety of sources would improve the precision and accuracy of genetically based diagnostics. Delivering diagnostic services by distributing the tasks involved would also improve the efficiency, timeliness, and quality of the services performed.

## Summary of the Invention

The invention is a method for providing clinical diagnostic services comprising analyzing the results obtained from testing of a biological sample to determine at least a part of the composition of its genetic material, the behavior of the genetic material, or a protein and incorporating information obtained through the analysis into subsequent analyses of biological samples. The results of the analysis can be reported to another party (e.g., to a health care provider).

Another aspect of the invention is a method for providing clinical diagnostic services that includes collecting a biological sample, analyzing the biological sample to determine at least a part of the composition of its genetic material, the behavior of the genetic material, or a protein, reporting the results of the analysis (e.g., to a health care provider), and incorporating information obtained through the analysis into subsequent analyses of biological samples. The information obtained from the analysis can, for example, be incorporated into subsequent analyses by using it to improve the algorithmic or database components of the information products used or can be used to improve the statistical reliability of the analyses.

The invention also includes systems for employing the method described above and articles of manufacture useful in such systems (e.g., computer readable media comprising the instructions for executing algorithms and manipulating databases).

## Brief Description of the Drawings

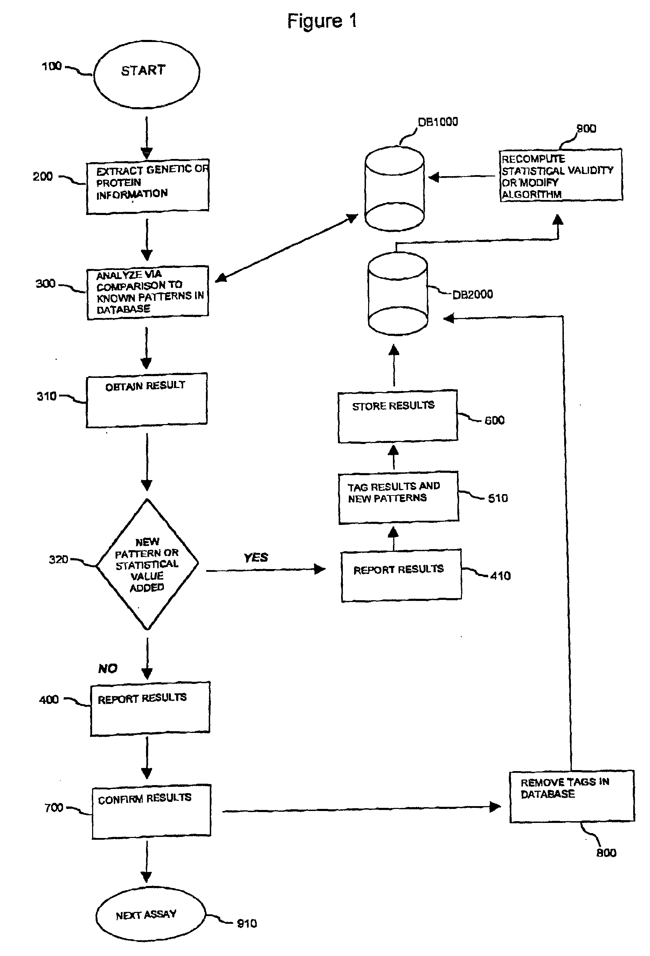

Fig. 1 is a flowchart illustrating a method of the invention.

## Detailed Description

Definitions:

The following terms are used throughout the specification.

"Internal database" means a database that contains biomolecular sequences (e.g., nucleotides and amino acids) to which a sample sequence or profile is compared. It may contain information associated with sequences such as the library in which a given sequence was found, descriptive information about a likely gene associated with the sequence, physiological manifestations associated with the sequence, and any other information helpful in associating sample sequence or the behavior of genetic material with condition or disease state. In addition, the database can contain patterns of gene expression characteristic of a cell or tissue type, patterns of DNA methylation that characteristic of cell or tissue type or any other heritable or somatically-derived genetic variation that are characteristic of cell or tissue types. The internal database employs sequence database components that are information indicative of the sequences of biomolecules that are embedded data structures or are found in discrete separate databases that accessed by the internal database as needed.

"Analytical Database" is a class of Internal database that is used as a reference in the process of determining some information about a cell or tissue that requires characterization. For example, it may be advantageous to determine whether cells or tissue removed from a patient exhibit characteristics of cells or tissues that require some form of medical intervention that could be beneficial to the host of the cell or tissue, This kind of analysis can be described as screening, diagnostic, prognostic or can be a monitoring procedure. A key feature of any analytical database is that the data contained therein is at least partially organized so that information of the subject can be compared against characterized references and conclusions can be made regarding the subject material with a predetermined level of confidence.

"Discovery database" is a class of internal database that contains sequence or pattern data collected from a wide range of sources. The discovery database is analyzed to identify sequences or patterns that could have utility as a component of an analytical database. Once a component of a discovery database reaches a determined level of significance, it is placed into an analytical database. This can occur according to preprogrammed rules. The discovery database has a level of order that allows multiple queries using multiple parameters either simultaneously or sequentially. Typically the data entered into a Discovery database will include genetic data annotated by clinical information. This mirrors the currently acceptable situation regarding patient privacy protection. For example, an entry to the database could be RNA expression profiles of a biopsy from a suspected prostate tumor where the expression data is electronically linked to a complete profile of the patient's medical history and current disease status. Mechanisms can be used in which later data about the patient is collected and added to the annotation fields for the pattern. The data describing the patient would be anonymous or coded and the entry into the database can be coded (e.g., using tags, described below in a different context). The code is sent to either the patient or physician and on representation the new data is sent attached to a code. The code allows the annotation to be lodged correctly. Only those individuals with the code, namely physician or patient will have access to the identifiable (with reference to the patient) data.

"Reference Pattern" or "Reference Sequence" are sequences or patterns that have been identified from within a discovery database and that have been shown to have diagnostic or prognostic utility. Reference sequences or Patterns are typically discovered in Discovery databases and then exported into the Analytic Database for use in medical practice. The flow of Reference materials is normally unidirectional from Discovery to Analytic Databases whereas the flow of sequences or patterns that have yet to be determined as whole or part of reference sequence or patterns can come from an entry into the Analytic database followed by export to the Discovery Database or they can be entered directly into the Discovery database.

"External database" means a database located outside the internal database. Typically, it is maintained by an enterprise that is different from the enterprise maintaining the internal database. In the context of this invention, the external database is used primarily to obtain information about the various sequences stored in the internal database. The external database may be used to provide some descriptive information stored in the gene expression database. In a preferred embodiment, the external database is GenBank and associated databases maintained by the National Center for Biotechnology Information (NCBI), part of the National Library of Medicine. GenPept is the associated public protein-sequence database that contains all the protein databases from GenBank. Other examples of external databases include the Blocks database maintained by the Fred Hutchinson Cancer Research Center in Seattle and the Swiss-Prot site maintained by the University of Geneva.

"Record" means an entry in a database table. Each record contains one or more fields or attributes. A given record may be uniquely specified by one or a combination of fields or attributes known as the record's primary key.

"Sequence" in the case of a nucleic acid, means one or more nucleotides that comprise the nucleic acid in the order in which they so comprise it. In the case of a protein, it means one or more amino acids that comprise the protein in the order in which they so comprise it.

"Pattern" means a sequence or group of sequences that form the basis of a comparison between known and sample genetic material or protein structure (e.g., amino acid sequence). Patterns can be the behavior of a group of gene sequences. For example, a pattern could be the relative gene expression activity of a set of defined genes where the observed behavior is characteristic or diagnostic of a specific physiological activity such as apoptosis or characteristic of the development of a disease. Furthermore the pattern of a relative gene expression levels could be indicative of the likely course of development of a cancer cell or cancerous tissue. Patterns of this type are sometimes referred to as cell or tumor profiles, genetic signatures or expression profiles, The act of determining patterns is therefore commonly referred to as profiling. Additionally, patterns may include other structural or behavioral identifying features of the genetic material such as epigenetic alterations. For example, patterns can be the status of DNA methylation of a group of genes. Methylation patterns could be the relative hyper or hypomethylation status of multiple genes and the methylation pattern can be characteristic or diagnostic of a specific physiological activity such as apoptosis or characteristic of the development of a disease. Furthermore the pattern of DNA methylation could be indicative of the likely course of development of a cancer cell or cancerous tissue. Patterns can also be groups of genetic changes or mutations such as groups of single nucleotide polymorphisms (SNPs). For example, where SNPs are reproducible seen to co-exist within an individual's genome and where there is confidence that these groups of SNPs are correlative and/or predictive these SNPs constitute a pattern. SNP Patterns can contain SNPs that are spaced throughout the genome or patterns of SNPs can form haplotypes where the co-inherited SNPs are in linkage disequilibrium. Patterns can also include conserved co-incidental events that may be drawn from any of the genetics events described above, for example, a pattern may include a SNP in a specific gene, a specific relative level of expression of 20 defined genes, a reproducible deletion of a chromosomal deletion (such as in Loss of Heterozygosity) and a hypermethylated region of defined chromosome. The defining feature that makes this collection of events a pattern is that they are predictive, diagnostic or prognostic of a gross phenotype or disease in the same individual harboring all of the genetic changes.

"Behavior" of genetic material means the way in which a sequence is manifested. In the case of nucleic acid sequences, the expression of a gene or sequence is one measure of the behavior of that sequence.

Sequence Analysis

Methods for determining nucleic acid sequences are now well known. Primary nucleotide sequencing can be completed by any number of methods including dideoxy termination sequencing. The analysis of the presence, absence or quantification of relative levels of RNA or DNA can be completed by many published methods including northern, Southern blotting, in situ hybridization, slot or dot blotting to name a subset of the entire repertoire. More recently, microarray technology has been used to determine whether various sequences are present and whether identified genes are being expressed. A few examples of such microarray technologies are found in U.S. Patents 6,004,755; 6,051,380; 5,837,832, each of which is incorporated herein by reference. These methods employ a substrate to which is bound a number of oligonucleotides that are typically labeled. When a sample containing a sequence that is complementary to the bound oligonucleotide is contacted with the substrate bound oligonucleotide, the method employs some form of signal to indicate that hybridization has occurred. For example, the solution-based molecule, typically the sample, can be labeled and the presence of the label detected by fluorescence microscopy or radiography. Alternatively, the two molecules bind and produce some detectable phenomena such as fluorescence. Microarray based methods can exploit a number of different technologies (e.g., some are passive, others are active) but they all have the potential to identify and characterize a number of sequences simultaneously. Other methods can also be used to analyze parallel numbers of sequences including cDNA sequencing. Serial Analysis of Gene Expression (SAGE) and the use of solution-based arrays in which specific oligonucleotides are linked to tagged beads. Following solution hybridization, the act of hybridization is detected by a range of published methods. Any method for determining the nucleic acid sequence can be used in the conjunction with the practice of this invention but the highly parallel methods described such as the microarray approach is most preferred. Methods for determining amino acid sequences are also well known.

To practice the methods of this invention, sequence information or gene expression profiles are obtained. At some point, therefore, a patient sample must be obtained. There are no limitations on the type of sample that can be used provided that the sample can be assayed to determine the sequence information. Thus, samples can be obtained from circulating blood, tissue biopsy, lavages, and any other method that will capture sequences. A panoply of methods for extracting such samples is available.

Sequence information can be produced and portrayed in a wide variety of methods. For example, where microarrays having bound fluorescently labeled oligonucleotides are used, a reader can be used to produce a graphic illustration of each bound sample oligonucleotides. These graphics can be digitized so that the intensity of each detectable event is measurable. This can be very useful in gene expression analysis where the determination of the production of RNA segments is an important indicator. Alternatively, one or more PCR reactions can be used to simply indicate whether particular segments are present. The information can then be cast in a table, database, or the like.

Any method of presenting sequence information or gene expression profiles can be used in the practice of this invention.

Bioinformatics.

As noted above, much of the diagnostic utility of bioinformatic systems is derived from the process of comparing or matching sample sequences or expression patterns with those of known sequences or known expression patterns. Various techniques may be employed for this purpose. Comparing structural data (e.g., genomic sequences) and expression data (e.g., gene expression profiles) can be done using the same or similar approaches since pattern matches between known and sample patterns is conducted. Using the nucleotide sequence data from patient samples as query sequences (sequences of a Sequence Listing), databases containing previously identified sequences can be searched for areas of homology (similarity). Examples of such databases include GenBank and EMBL.

One homology search algorithm that can be used is the algorithm described in the paper by D. J. Lipman and W. R. Pearson, entitled "Rapid and Sensitive Protein Similarity Scarches", Science, 227, 1435 (1985). In this algorithm, the homologous regions are searched in a two-step manner. In the first step, the highest homologous regions are determined by calculating a matching score using a homology score table. The parameter "Ktup" is used in this step to establish the minimum window size to be shifted for comparing two sequences. Ktup also sets the number of bases that must match to extract the highest homologous region among the sequences. In this step, no insertions or deletions are applied and the homology is displayed as an initial (INIT) value. In the second step, the homologous regions are aligned to obtain the highest matching score by inserting a gap in order to add a probable deleted portion. The matching score obtained in the first step is recalculated using the homology score Table and the insertion score Table to an optimized (OPT) value in the final output.

DNA homologies between two sequences can be examined graphically using the Harr method of constructing dot matrix homology plots (Needleman, S. B. and Wunsch, C. O., J. Mol. Biol 48:443 (1970)). This method produces a two-dimensional plot that can be useful in determining regions of homology versus regions of repetition.

However, in a class of preferred embodiments, the comparison between nucleic acid sequence and expression data obtained from samples and the reference pattern is implemented by processing the data obtained from patient sample in the commercially available computer program known as the INHERIT 670 Sequence Analysis System, available from Applied Biosystems Inc. (of Foster City, Calif.), including the software known as the Factura software (also available from Applied Biosystems Inc.). The Factura program preprocesses each sample sequence to "edit out" portions that are not likely to be of interest such as the polyA tail and repetitive GAG and CCC sequences. A low-end search program can be written to mask out such "low-information" sequences, or programs such as BLAST can ignore the low-information sequences.

In the algorithm implemented by the INHERIT 670 Sequence Analysis System, the Pattern Specification Language (developed by TRW Inc.) is used to determine regions of homology. "There are three parameters that determine how INHERIT analysis runs sequence comparisons: window size, window offset and error tolerance. Window size specifies the length of the segments into which the query sequence is subdivided. Window offset specifies where to start the next segment [to be compared], counting from the beginning of the previous segment. Error tolerance specifies the total number of insertions, deletions and/or substitutions that are tolerated over the specified word length. Error tolerance may be set to any integer between 0 and 6. The default settings are window tolerance=20, window offset=10 and error tolerance=3." INHERIT Analysis Users Manual. pp. 2-15. Version 1.0. Applied Biosystems. Inc. October. 1991. Using a combination of these three parameters, a database can be searched for sequences containing regions of homology and the appropriate sequences are scored with an initial value. Subsequently, these homologous regions are examined using dot matrix homology plots to determine regions of homology versus regions of repetition. Smith-Waterman alignments can be used to display the results of the homology search. The INHERIT software can be executed by a Sun computer system programmed with the UNIX operating system.

Search alternatives to INHERIT include the BLAST program, GCG (available from the Genetics Computer Group, WI) and the Dasher program (Temple Smith, Boston University, Boston. MA). Nucleotide sequences can be searched against GenBank, EMBL or custom Internal Databases such as GENESEQ (available from Intelligeneties, Mountain View, CA) or other Internal Databases for genes.

The BLAST (Basic Local Alignment Search Tool) program and the Smith-Waterman algorithm look for regions of ungapped similarity between two sequences. To do this, they determine (1) alignment between similar regions of the two sequences, and (2) a percent identity between sequences. The alignment is calculated by matching, base-by-base, the regions of substantial similarity. In these regions, identical bases are scored with a value of +5 and mismatched bases are scored with a value of -4 (for nucleic acids). Regions of contiguous bases having sufficiently high score are deemed High Scoring Pairs ("HSPs"). In BLAST, the score of the best HSP (referred to as the BLAST Score) is presented as an output. In addition, for each HSP, the percent identity is calculated and presented as a BLAST output, as is the alignment. Finally, a P-Value for each HSP is calculated. The P-Value represents the probability that the observed similarity resulted from a random occurrence. Lower P-Values indicate greater confidence that the observed similarity is not due to a random event.

The Product Score represents a normalized summary of the BLAST output parameters and is used to represent the quality of an alignment between a query and matched sequence. Specifically, the Product Score is a normalized value between indicating the strength of a BLAST match; it represents a balance between fractional overlap and quality in a BLAST alignment.

Numerous other sequence matching/analysis algorithms are available. The FASTA method, for example, first compares the largest number of short perfect matches of sequences in a process referred to as hashing. The best-matched sequences are then subjected to a second analysis that scores the match according to separate criteria than that used in the first comparison. Finally, the best-matched sequences are aligned and provided with a score based on parameters relating to the closeness of the alignment.

In one aspect of this invention, matching algorithms and associated databases can comprise a portion of the system used to arrive at a diagnosis, prognosis, or staging of a condition or disease state. Another aspect of the system is an internal database that is continuously updated so that sequences assessed during the analysis of each sample are incorporated into the analytical database that is used to compare subsequent sample sequences. That is, sequences generated from patient sample analyses are later incorporated into reference patterns.

The database that is used to match patient sample nucleic acid sequences or gene expression profiles with known sequences or profiles further correlates those sequences with clinical results to ascribe clinical meaning to the identified sequences. These correlations can be stored and manipulated from the same database used to determine homology or they can be stored and maintained in a separate database to which the homology determining database and algorithm are interfaced. By way of example, nucleic acid sequences indicative of amplification of the her-2-neu gene in conjunction with the presence or absence of other as yet undiscovered nucleic acid sequences may indicate that the patient is developing aggressive breast cancer. Likewise, enhanced expression or greatly reduced expression of a gene may also indicate uncontrolled growth of a cell type. Once homology or pattern similarity is established between these sequences or gene expression profiles and those of the patient sample, the sequences or profiles are matched with the clinical meanings ascribed to them in the analytical database. A clinical result (i.e.. information) is then generated indicating, in the case of the her-2-neu gene, that the patient is developing aggressive breast cancer.

Establishing gene expression profiles is conducted through a process such as the following that would be useful for predicting whether a patient previously identified with a tumor will relapse. A class prediction model is established in which (1) a discriminating relationship is defined (e.g., relapse v. survivor), (2) scoring individual genes for their ability to predict the desired pattern and evaluation of the statistical significance of these scores, (3) selection of a subset of informative genes, (4) construction of a prediction rule based on this subset, and (5) validation of the rule on the initial data set and on independent data. Such schemes have been successful in analyzing data from a wide range of tumors. The methods typically vary in the selection of scores, the calculation of significance and the exact method of rule construction.

In order to select particular gene expression markers, each gene on a microarray of genes indicative of or associated with cancer are scored according to the "similarity" of each such gene with the desired discrimination of the two classes. Different distances and measures can be employed as such scores. From that process, a list of genes are produced and further narrowed according to additional considerations in order to produce a signature subset.

Predictors are constructed from the narrowed list of signature subsets. In the predictor, each of the genes casts a weighted vote for one of the classes (relapse or survivor) and the class with more votes (above a given victory margin) wins the prediction. The weight of each gene's vote depends on its expression level in the new sample and its "quality" as reflected by its score. The votes for each class are summed and compared to determine the winning class, as well as a prediction strength that is a measure of the margin of victory. Samples are assigned to a winning class only if the prediction strength exceeds a given pre-set threshold.

Predictors are cross-validated and evaluated preferably in conjunction with an independent data set, since most classification methods will work well on the examples that were used in their establishment. Samples can be divided into 2 or more groups for validation. Or a commonly used method of cross-validation, such as Leave-One-Out Cross Validation (LOOCV can be used. Multivariant analysis can then be applied to test association between patient prognosis data and marker expression assessed.

An exemplary method for comparing expression information follows: Labeled cDNA molecules are hybridized to a microarray containing complementary nucleic acid sequences and a label (e.g.. with fluorophor). The microarray is then scanned and the intensity of the spots are recorded. A matrix of the intensity data is then prepared.

A reference gene expression vector is then prepared. If A, B, ... Z are used to denote the groups of samples to be differentiated, $a, b, ... z$ are used to denote the number of samples used to construct the reference gene within each group respectively. Thus, the notation $A_{21}$ represents the expression intensity from the 2nd gene in sample 1 of group A. If each sample was hybridized onto a microarray with size $n$ genes, then the following matrices A, B, ... Z represent expression data from all of the groups A, B, ... Z respectively.

$$\begin{bmatrix} A_{11} & A_{12} & \cdots & A_{1a} \\ A_{21} & A_{22} & \cdots & A_{2a} \\ \vdots & \cdots & \ddots & \vdots \\ A_{n1} & A_{n2} & \cdots & A_{na} \end{bmatrix} \begin{bmatrix} B_{11} & B_{12} & \cdots & B_{1b} \\ B_{21} & B_{22} & \cdots & B_{2b} \\ \vdots & \cdots & \ddots & \vdots \\ B_{n1} & B_{n2} & \cdots & B_{nb} \end{bmatrix} \cdots \begin{bmatrix} Z_{11} & Z_{12} & \cdots & Z_{1z} \\ Z_{21} & Z_{22} & \cdots & Z_{2z} \\ \vdots & \cdots & \ddots & \vdots \\ Z_{n1} & Z_{n2} & \cdots & Z_{nz} \end{bmatrix}$$

The geometric mean expression value for each gene in each matrix is then calculated so that the following matrixes are prepared (if $A_{1(geomean)}$ is the geometric mean of set $\{A_{11} A_{12} \cdots A_{1\alpha}\}$, gene 1 in group A).

$$\begin{bmatrix} A_{1(geomean)} \\ A_{2(geomean)} \\ \vdots \\ A_{n(geomean)} \end{bmatrix} \begin{bmatrix} B_{1(geomean)} \\ B_{2(geomean)} \\ \vdots \\ B_{n(geomean)} \end{bmatrix} \cdots \begin{bmatrix} Z_{1(geomean)} \\ Z_{2(geomean)} \\ \vdots \\ Z_{n(geomean)} \end{bmatrix}$$

The reference gene expression vector is the geometric mean of those vectors.

$$\begin{bmatrix} \bar{X}_1 \\ \bar{X}_2 \\ \vdots \\ \bar{X}_n \end{bmatrix}$$

where $\bar{X}_1$ is the geometric mean of $\{A_{1(geomean)} B_{1(geomean)} \cdots Z_{1(geomean)}\}$

After the reference gene expression vector is prepared, the original data set is transformed by taking the log of the ratio relative to the reference gene expression value for each gene. This produces matrixes $\{A' B' \cdots Z'\}$.

$$\begin{bmatrix} A'_{11} & A'_{12} & \cdots & A'_{1a} \\ A'_{21} & A'_{22} & \cdots & A'_{2a} \\ \vdots & \cdots & \ddots & \vdots \\ A'_{n1} & A'_{n2} & \cdots & A'_{na} \end{bmatrix} \begin{bmatrix} B'_{11} & B'_{12} & \cdots & B'_{1b} \\ B'_{21} & B'_{22} & \cdots & B'_{2b} \\ \vdots & \cdots & \ddots & \vdots \\ B'_{n1} & B'_{n2} & \cdots & B'_{nb} \end{bmatrix} \cdots \begin{bmatrix} Z'_{11} & Z'_{12} & \cdots & Z'_{1z} \\ Z'_{21} & Z'_{22} & \cdots & Z'_{2z} \\ \vdots & \cdots & \ddots & \vdots \\ Z'_{n1} & Z'_{n2} & \cdots & Z'_{nz} \end{bmatrix}$$

where $A'_{11} = \ln(A_{11} / \bar{X}_1)$ and $Z'_{nz} = \ln(Z_{nz} / \bar{X}_n)$. The values then represent fold increase or decrease over the average for each gene.

Genes with weak differentiation power are then removed from matrixes $\{A'\ B'\ \cdots\ Z'\}$. For gene $i$ from $l$ to $n$, gene $i$ is removed from all the matrices if none of its values $\{A'_{i1}, A'_{i2}, \cdots A'_{ia}, B'_{i1}, B'_{i2}, \cdots B'_{ib}. Z'_{i1}, Z'_{i2}, \cdots Z'_{iz}\}$ in absolute number is greater than a threshold value (ln3 in the preferred embodiment). In other words, to be considered a diagnostically relevant gene, the value must have at least one value in any matrix with absolute value greater than or equal to the threshold value (ln3, preferably). Matrixes with genes having weak differentiation power removed are now matrixes $\{A''\ B''\ \cdots\ Z''\}$.

A signature extraction algorithm is then applied to each resulting matrix $\{A''\ B''\ \cdots\ Z''\}$, to create a signature as follows. The algorithm in this case is referred to as the Maxcor algorithm and works on each group $\{A''\ B''\ \cdots\ Z''\}$ separately. For each pair of columns in the matrix, the genes coordinately expressed in high, average, and low over the mean (defined below) are given a value 1, 0, and -1 respectively producing a weight vector representing the pair. For matrix A", $\frac{a(a-1)}{2}$ pairwise calculations are performed, A final average weight vector, referred to as the signature for group A, is calculated by taking the average of all $\frac{a(a-1)}{2}$ weight vectors from matrix $A''$. Thus, the signature contains the same number of genes as $A''$ and its values should be within [-1,1] with -1 and 1 representing genes consistently expressed in low and high levels relative to the mean of all the groups respectively.

The pairwise calculations referred to above are conducted by taking coordinate columns c1 and c2 and normalizing their values such that, $c1_i$ became $\frac{c1_i - \bar{c}1}{S_{c1}}$ where $\bar{c}1$ is the mean of column c1 and $S_{c1}$ is the standard deviation. For each gene pair in $c1'$ and $c2'$, the product is then stored in vector $p12$ with each value in $p12$ then being sorted from lowest to highest. A nominal cutoff value ( 0.5 in the preferred embodiment) is then used to collect all genes with a greater product value in $p12$. The Pearson correlation coefficient for this set of genes using values in column c1 and c2 is then calculated. The cutoff value is then increased until the correlation coefficient is greater than a statistically relevant number (0.8 in the preferred embodiment). When this is completed, the set of genes meeting this criteria is assigned 1 if both gene values in $c1'$ and $c2'$ are positive, -1 if both gene values are negative. For all other genes in $c1'$ and $c2'$, 0 is assigned. The resulting vector is the weight vector representing the pair. The -1 and 1 values represent the genes consistently expressed in low or high levels, respectively, relative to the mean of all groups.

Once a signature is prepared, unknown samples can then be scored against it. Before scoring, the genes in sample $S$ with weak differentiation value are removed so that the rows remaining are the same as those in the signature vectors. thus creating sample vector $S''$. The score is the sum of the products for each gene in $S''$ and its weight in the signature vector. For example, the score between sample vector $S''$ and signature vector $A^s$ is $\sum_{i=1-n} S''_i\ A^x_i$. The normalized score is (score - mean of randomized score)/standard deviation of randomized score, where randomized score is the score between $S''$ and the signature vector which has its gene positions randomized. Typically 100 randomized scores are generated to calculate the mean and the standard deviation. A high score indicates that the unknown sample contains or is related to the sample from which the signature was derived.

Alternative signature extraction algorithms can also be used. One example is the Mean Log Ratio approach. This algorithm works on each group/matrix $\{A''\ B''\ \cdots\ Z''\}$ separately.

For each matrix, the signature vector is the row mean of the matrix. Thus, the signature vectors for groups $\{A''\ B''\ \cdots\ Z''\}$ are:

$$\begin{bmatrix} \bar{A}''_1 \\ \bar{A}''_2 \\ \vdots \\ \bar{A}''_n \end{bmatrix} \begin{bmatrix} \bar{B}''_1 \\ \bar{B}''_2 \\ \vdots \\ \bar{B}''_n \end{bmatrix} \cdots \begin{bmatrix} \bar{Z}''_1 \\ \bar{Z}''_2 \\ \vdots \\ \bar{Z}''_n \end{bmatrix}$$

where $\bar{A}''_1$ is the mean of $\{A''_{11}, A''_{12}, \cdots A''_{1a}\}$.

Scoring an unknown sample using this approach is conducted as follows. Before scoring, the sample gene expression vector is transformed by taking the log of the ratio relative to the reference gene expression vector created. For example, transformation of sample

$$S = \begin{bmatrix} S_1 \\ S_2 \\ \vdots \\ S_n \end{bmatrix}$$

leads to

$$S' = \begin{bmatrix} S'_1 \\ S'_2 \\ \vdots \\ S'_n \end{bmatrix},$$

where $S'_1 = \ln(S_1/\bar{X}_1)$.

Next, genes with weak differentiation value are removed so the rows remaining are the same as those in the signature vectors, thus creating sample vector $S''$. The score against each signature is then calculated by taking the Euclidean distance between $S''$ and the signature vector. The normalized score is (score - mean of randomized score) /standard deviation of randomized score. where randomized score is the Euclidean distance between $S''$ and the signature vector which has its gene positions randomized.

The patient data can also be used to improve the database(s) and the algorithms used to conduct the operations described above. Databases are improved by incorporating information about patient sequences or patterns from a discovery database into an analytical database. This improves the statistical reliability of the matching process (between clinical meaning and sequence) by increasing sample size. This is true whether the sequence or pattern is reported as indicative of a negative or positive clinical result provided that the result is correct. Additionally, some samples will have sequences or patterns that were not present in the sequences or patterns in the database with which they were compared. These sequences or patterns can provide additional characteristics that will strengthen matches when future samples having the same sequence profile are analyzed.

Whether additional confidence can be attained through the use of additional pattern matching is also considered. That is, different levels of confidence may be ascribed to matches with different patterns. Thus, while the minimum pattern match may have been established to arrive at a particular diagnosis, the presence or absence of further matches that would be considered superfluous under the Daimond model (described below) can be used to improve the confidence in the results.

U.S. patent 5,692,220 to Diamond proposes a simple set of questions when considering whether to include a given pattern in an algorithm. He asks first what minimum set of input data must be present to establish a positive match with the pattern under consideration? Next, he asks whether there is any single piece of input data, or combination of input data, which, when present, rules out, i.e., excludes, that pattern from further consideration? Finally, he asks whether other patterns already programmed for comparison are lower on the hierarchy than the pattern being considered. That is, whether other patterns can be "swallowed" by the pattern under consideration?

In the instant invention, the last two questions are answered as part of the process for determining whether and how algorithms correlating clinical meaning with sequence information should be modified. Under the Diamond model, if a pattern could be swallowed by another pattern. one would then use the broader pattern. However, where additional confidence can be attained by attributing higher scores to data that matched across more patterns, it would be valuahle to retain the use of both patterns. The same can be said about considering whether or not to use a single, apparently definitive match, as opposed to a number of pattern matches. The Diamond model suggests only using the single match if possible. However, in the instant case this may not be desirable if greater, statistically significant, confidence can be attained through the use of multiple points of comparison.

Fig. 1 is a flowchart illustrating a method of incorporating expression profile data into the diagnostic/prognostic algorithms to enhance confidence. The statistical tools for calculating confidence level, appropriate sample size, and like considerations are all well known. Programming the methods into executable computer code is also conventional and readily achieved by any person skilled in the art of computer programming. The act of conducting this process as a continuous and/or preprogrammed process in conjunction with processing patient data is an aspect of the inventive method. This exemplary process is started in Step 100 by a health care provider or other relevant party requesting an analysis of patient sample. In Step 200, the sample has been obtained and the physical manipulative steps of conducting the laboratory assay is conducted either by the health care provider, a laboratory service, or the party that operates the database system. The culmination of this step is the extraction of genetic material or protein material from which sequence information is derived. This information is then analyzed in Step 300 via comparison with reference sequences and interrogation via algorithms. The reference sequences are stored in analytical database 1000. The algorithms used to conduct the analysis can be conducted as part of the programming instructions in database 1000 or they can be operated via a separate series of instructions in an independent computer program made to query and manipulate database 1000. Analysis in Step 300 generates a result. Step 310. This result will indicate if there is a match with a reference pattern sufficient to provide a diagnosis, prognosis, or other clinically relevant information. The system is queried to determine whether the matching process identified any patterns not previously identified or whether the identification of a previously identified pattern (or its absence) in this sample would provide additional statistical value, step 320. Additional statistical value can be obtained, for example. by increasing sample size such that increased confidence or predictive power is attained. Results are reported in Step 400 or Step 410 to the party that requested them or where such results were designated to be sent. The result can be communicated directly to the health care provider via electronic communication or in any other way. The patterns are tagged if they present patterns not previously identified as having clinical significance, or which will be the more usual case, when a pattern emerges that has been previously identified as being potentially relevant to a clinical state but where sufficient confidence in the relationship has not yet been established. This tagging occurs in Step 510. The tagged pattern is stored in the discovery database DB 2000 in Step 600. Upon receiving confirmation of clinical state from the health care provider (Step 700) or other who is in a position to provide it, the tag is removed from the data (Step 800). The pattern is then moved from the discovery database 2000 and moved into analytical database 1000 to be used as a reference signature in subsequent analyses. The process can be iterative if, for example, more than one new pattern is identified by the pattern matching algorithm and different portions of the patterns correlate with different clinical information that requires separate confirmation.

The process of this invention is not dependent upon the establishment of normal ranges in the same sense as those used in ANNs and standard diagnostic methods found in the prior art (such as clinical chemistry and EIA assays). In the case of single or definitive nucleic acid or protein patterns indicative of disease state or condition, any presence of the marker (e.g., gene) has clinical meaning. On the other hand, where combinations of markers are used to establish a clinical diagnosis or statistical confidence is attributed to a group of markers, the patterns to which unkowns or samples are compared can change continuously. To the extent that one might view a pattern as a "normal" it is a dynamic normal unlike normals ordinarily associated with analytes measured in classical diagnostic medicine. The normal is constantly updated and validated.

The addition of patterns from patient samples into the database and algorithms of the refernce patterns of the analytical database presents some challenging issues. How, for example, does one know when a pattern that has not been previously seen can be used to bolster a diagnosis, weaken the confidence in a diagnosis, or suggest a diagnosis not previously determinable? In the most preferred embodiment of the invention, upon initial analysis, sequences that are matched against a database are provided with some indicia (e.g., they are "tagged" with a data element") indicating that the diagnosis has not been independently confirmed. In this most preferred embodiment, the tagged sequence resides in the discovery database. Suppose that a sample displays a sequence that has a match with a known pattern but also displays a pattern that has not yet been correlated to a disease state or physical condition. Independently, other similar patterns containing a mix of known and previously unknown patterns are conducted. A result based on matches with previously identified patterns is reported but the previously unknown pattern is not yet incorporated into the process of analyzing subsequent sample sequences. The tagged data can be assigned to a data table or database (e.g., discovery database). Upon receiving information that confirms the physical condition or disease state and upon establishment of the association of the previously unknown pattern with a given clinical condition, the indicia ("tag") is removed and the sequence becomes fully incorporated into the matching process or becomes incorporated into the statistical values that drive the matching algorithm. An internal register can be used to ascribe statistical significance to the newly added pattern. That is, the first such "confirmation" of the simultaneous appearance of the pattern and independent confirmation of disease state may be assigned a value or given a notation indicating that the pattern is suspected of relating to a given diagnosis. When the pattern is seen again and it is correlated to the presence of a disease or condition it is given a different indictor, such as one that means that the disease state or physical condition is likely. This course can be followed until the correlation between the presence of the pattern and disease state or condition is well established according to well known statistical methods and standards.

In terms of databases, this process can be implemented as follows:

1. A large set of characterized patient samples are treated so that sequences or patterns are identified. For example, a large collection of approximately 200 to 400 samples representing two distinct cell or tissue types would be collected and the sequence or pattern data is placed into a Discovery database. The Discovery database is analyzed using bioinformatic methods until a pattern is detected that discriminates between two or more different types of cells or tissues in such a way where that data is useful.

2. The data set required to define the full range of patterns related to the variable of interest is exported to an Analytical Database. This database is "locked" and used as a clinical reference tool for clinical diagnosis of patients.

3. The diagnosis operates by analyzing new patients with a device designed to measure the predetermined patterns. The new data is compared against the Analytic Database and a statistical assessment is made on similarity between the patient sample and a reference pattern.

4. At the same time, the patient pattern is inserted into the Discovery Database. The new data is combined with all the preceding data. During each periodic review of the discovery database for new patterns, the newly submitted patterns are included in the new data set. In time, the statistical value of the discovery set increases and the statistical power of the reference patterns increases.

5. At each point that the reference patterns are derived from the discovery database and they are statistically superior to preceding patterns, the new patterns replace the Analytic Database and act as reference patterns.

In a preferred embodiment, the interface between the Discovery and Analytic database is "live". In this case there is no physical separation of the two databases but the Analytic domain is defined as a subset within the discovery database. The method of analyzing the discovery database and updating the analytic database reference patterns is continuous.

An important variation on the method is a case in which there are several discovery databases focusing on different patterns. For example, separate discovery Databases can focus on cancers of different organs. As well as shuffling data from constantly improving Discovery databases to respective Analytic Databases, the separate databases can be merged to form one large discovery database. With the combination of multiple patterns, particularly where they are annotated with information concerning related and unrelated phenotypic features, entirely new patterns that are useful references for new phenotypes can emerge.

The tagging/untagging process can be accomplished in numerous ways. It is possible to manually affect the tagging and/or untagging process through an appropriate digitized command. For example, when informing the recipient of the analysis, the recipient could be advised that they should inform the database operator of the clinical diagnosis when it is confirmed through a means distinct from genetic testing (e.g., biopsy and cell analysis). Where the requester is in electronic communication with the provider of the analysis, a simple connection can be created so that requester inputs confirmatory data directly into the database thus removing the tag. Of course, consideration must be given to circumstances in which confirmation of the analysis cannot be made. In such a case, the tagged data can remain tagged, can be discarded, or can be used to affect the statistical reporting associated with the analysis (e.g., it can be used to lower the confidence in the result). Implementing any of these options is a simple matter from a programming perspective and is readily achievable by one of ordinary skill.

Preferred Embodiments

The methods of this invention can be practiced in many different manners. There are many combinations of sample collection, analysis, reporting, data collection, database, and analysis improvement processes. The most preferred combinations are those that match the best capabilities of the various parties involved with the functions that require those capabilities. Additionally, efficiency is a consideration. It is most efficient that the analysis process be conducted at one or a few centralized locations given the requirements associated with storing and manipulating large databases with sophisticated algorithms that are being continuously improved in the manner described above. This eases hardware and software maintenance and upgrade concerns, and most importantly limits requirements associated with distributing the improvements to the algorithms and databases. Likewise, sample testing (i.e., the actual laboratory steps) to obtain the pattern may be best done at a local hospital or reference lab since such operations are generally best configured and staffed to conduct these activities.

In the most preferred method, a health care provider obtains a patient sample in the appropriate format. This will differ depending upon the suspected disease or condition. For example, if testing for breast cancer, a biopsy sample of breast tissue may be the appropriate sample whereas if testing is a general screening, a whole blood sample may be best. In any event, selection of the appropriate sample would be apparent to one of ordinary skill in the art and would be dependent upon by the assay format choices available.

After collecting the sample, the health care provider sends the sample under the appropriate conditions (e. g., in a tube containing the appropriate preservatives and additives) to a laboratory capable of obtaining the pattern needed for analysis using the bioinformatic system described herein. Preferably, but not necessarily, the assay for obtaining this pattern is provided by the same party and comprises a nucleic acid or protein microarray. Such devices are now well known. Their use is described in numerous patents such as: U.S. Pat. Nos. 5,143,854; 5,288,644; 5,324,633; 5,432,049; 5,470,710; 5,492,806; 5,503,980; 5,510,270; 5,525,464; 5,547,839; 5,580,732; 5,661,028; 5,848,659; and 5,874,219; the disclosures of which are herein incorporated by reference. Preferably. the data format is a digital representation of the pattern. This lends itself to additional formatting in Gene Expression Markup Language (GEML™, Rosetta Inpharmatics, Kirkland, Washington). This language is a published. documented, open format that enables interchange among gene expression systems, databases, and tools. Moreover, the format permits an unlimited number of tags. *C.f., Gene Expression Markup Languagage (GEML™). A Common Data Format for Gene Expression Data and Annotation Interchange*. Rosetta Inpharmatics, www.geml.org/docs/GEML.pdf (2000). This facilitates tagging data for later confirmation of clinical results and for rendering data anonymous as each is described *infra*.

The pattern obtained is provided in any input form (e.g., scanned into computer that can digitized the pattern) and then analyzed by the operator of the bioinformatic system. The results of the analysis (sequence/pattern match with predicted diagnosis or condition) are then communicated to the requester. At the same time, the pattern is tentatively held in the database associated with the bioinformatic system. Preferably, it is tagged as tentative as described above and retained in the discovery database. The requester then returns confirmatory information to the operator of the bioinformatic system. If confirmation is possible, the pattern and any new information that can be gleaned from the pattern becomes a part of the analytical database as a reference sequence. In some instances this occurs simultaneously since receipt of expression data confirms the diagnosis of the heath care provider who has already conducted other clinical evaluations. If nothing else were done with the data, the statistical reliability of the analysis will have been improved through increased sample size. The database will have been made more robust.

In another preferred embodiment a laboratory or health care provider obtains the required sample. The sample is assayed by the same organization as the one conducting the analysis. This has some advantage since the assay format and desired input format for the analysis can be more easily coordinated. The analysis of the patterns discerned and data/algorithmic improvements described above can then be conducted in similar fashion.

In any method in which the pattern to be analyzed must be communicated to a different location (e.g., where a laboratory conducts the assay and sends the pattern obtained to the bioinformatics operators), it is possible to employ electronic communication to quicken the process. The Internet and other networked systems can readily be employed to this end as will be appreciated by one of ordinary skill in the art.

The devices of this invention are best made and used when configured as specially programmed general use computers. In this embodiment, the database system (combination of discovery and analytical databases together with programming instructions to function as described above) performs its functions by a combination of one or more computers specially programmed to perform the functions described herein. The instructions can be incorporated into any suitable media for performing computer operations such as hard-drive, network, optical or magneto-optical material, and any others typically used for this purpose. Article of manufacture comprising media that is recorded with computer instructions for implementing the process described herein are a further embodiment of the invention.

## Claims

1. A method for providing clinical diagnostic services comprising:

    a) collecting a biological sample,
    b) analyzing said biological sample to determine at least a part of the composition of its genetic material, the behaviour thereof, or a protein.
    c) reporting the results of the analysis of said biological sample, and
    d) incorporating information obtained through the analysis of said biological sample into subsequent analyses of biological samples.

2. The method of claim 1 including the step of extracting genetic material from said biological sample.

3. The method of claim 1 including the step of extracting protein from said biological sample.

4. The method of claim 2 wherein the collection of biological sample and the extraction of genetic material from said biological sample is conducted by a laboratory service or health care provider and the analysis to determine the composition or behaviour of genetic materials and the incorporation of such information in subsequent analyses is conducted by an entity that is not the laboratory service or health care provider that conducted the collection and extraction steps.

5. The method of claim 3 wherein the collection of biological sample and the extraction of protein from said biological sample is conducted by a laboratory service or health care provider and the analysis to determine the composition, concentration, or behaviour of said protein and the incorporation of such information in subsequent analyses is conducted by an entity that is not the laboratory service or health care provider that conducted the collection and extraction steps.

6. The method of claim 2 further comprising the step of amplifying at least a portion of the genetic material.

7. The method of claim 2 wherein said analysing step is done in conjunction with a microarray.

8. The method of claim 2 wherein the collection and extraction steps are conducted by a laboratory service or health care provider and the analysis to determine the composition or behaviour of genetic materials and the incorporation of such information in subsequent analyses is conducted by an entity that is not the laboratory service or health care provider that conducted the collection and extraction steps.

9. The method of claim 1 wherein the step of incorporating information into the subsequent analyses of biological samples modifies the statistical validity of the results of the analysis.

10. The method of claim 1 wherein said analysis is conducted by comparing said genetic material, the behaviour thereof, or said protein with a database comprising pattern information; and wherein the step of incorporating information into the subsequent analyses of biological samples modifies the database.

# Figure 1